# EUROPEAN PATENT APPLICATION

(11) **EP 1 541 586 A1**
(43) Date of publication of application: **15.06.2005**
(21) Application number: 03741500.7
(22) Date of filing: 18.07.2003
(51) Int. Cl.: C07K 14/47, C12N 15/12, C12N 5/10

(54) **NOVEL GENE ASSOCIATED WITH RHEUMATOID ARTHRITIS**

(30) Priority: 22.07.2002 JP 2002211951
(71) Applicant: YAMANOUCHI PHARMACEUTICAL CO. LTD., Tokyo 103-8411 (JP); Harigai, Masayoshi, Itabashi-ku, Tokyo 173-0004 (JP)
(72) Inventor: TAKEUCHI, Masahiro, c/o Yamanouchi Pharma. Co Ltd., Tsukuba-shi, Ibaraki 305-8585 (JP); YAMAJI, Noboru, c/o Yamanouchi Pharma. Co. Ltd., Tsukuba-shi, Ibaraki 305-8585 (JP); TAKASAKI, Jun, c/o Yamanouchi Pharma. Co., Ltd., Tsukuba-shi, Ibaraki 305-8585 (JP); AKAMATSU, Masahiko, c/o Yamanouchi Pharma. Co Ltd., Tsukuba-shi, Ibaraki 305-8585 (JP); TSUNOYAMA, Kazuhisa, c/o Yamanouchi Pharma. Co Ltd, Tsukuba-shi, Ibaraki 305-8585 (JP); HARIGAI, Masayoshi, Itabashi-ku, Tokyo 173-0004 (JP)
(74) Representative: Bates, Philip Ian
(86) International application number: PCT/JP2003/009180
(87) International publication number: WO 2004/009626

(57) **Abstract**

It is disclosed that novel polypeptides which has enhanced expression levels in the synovial tissues of human chronic rheumatoid arthritis (RA) patients compared with the synovial tissues of human osteoarthritis (OA) patients, polynucleotides encoding the polypeptides, vectors comprising the polynucleotides, transformant cells comprising the vectors and an antibody against the polypeptide. It is also disclosed that polynucleotides specifically hybridizing with the polynucleotides and having at least 15 nucleotides, a detection method which is useful for RA diagnosis, and a detection kit which is useful for RA diagnosis.

## Description

### Technical Field

The present invention relates to a novel polypeptide relating to chronic rheumatoid arthritis (RA), a polynucleotide encoding the polypeptide, vectors comprising the polynucleotide, a transformant cell comprising the vector and an useful method for detecting RA diagnosis.

### Background of the Invention

RA is a chronic inflammatory disease of unknown origin, which has the mainlocus of lesion in the synovial tissue and causes flare, swelling, heat sensation, pain, movement restriction and destruction of joints. Overproduction of inflammatory cytokines such as interleukin-1 (IL-1), interleukin-6 (IL-6), interleukin-8 (IL-8), interleukin-12 (IL-12), interleukin-15 (IL-15), interleukin-18 (IL-18), tumor necrosis factor-α (TNF-α) and the like, nitric oxide (NO), prostaglandins (PGs) and the like is known in the synovial tissue of RA (cf. non-patent reference 1). Additionally, immunocytes which constitute the synovial tissue activate each other through molecules on cellular surface, for example CD40/CD40 ligand systems and ICAM-1 (intercellular adhesion molecule-1)/LFA-1 (leukocyte adhesion molecule-1) systems and relate to the prolongation of inflammatory reaction (see non-patent reference 2). In recent years, a therapeutic method aimed at IL-1, IL-6 or TNF-α has been developed using a monoclonal antibody and a soluble receptor, and its efficacy is drawing attention (cf. non-patent reference 3). However, there is a group of patients in which complete remission cannot be introduced by the conventional therapeutic method which uses a therapeutic target molecule as the mechanism (cf. non-patent reference 4). Accordingly, identification of a new therapeutic target molecule which is different from the already known reports is expected.

According to the progress of the analysis of the pathology of RA, the meaning of RA diagnosis at early stage and the meaning of its early therapy have been drawing attention, together with a challenge of a therapeutic method for suppressing of excessive synovial proliferation to suppress or prolong joint bone destruciton, using synovial inflammation and immune abnormality as targets. On the other hand, a certain university in USA proposes a criterion concerning RA classification (see non-patent reference 5). Since the criterion is a simple standard and RA includes highly various pathologic patterns, RA diagnosis, especially quantitative and simple diagnosis of RA has been considered to be much difficult. Accordingly, a quantitative and simple method for diagnosis of RA has been strongly desired.

The sequence which relates to the sequence of RA2 as one of the polypeptides of the present invention is registered as AK094461 (2002. 7. 15) on the database genpept and is described in the patent reference 1, the patent reference 2 and the patent reference 3. In the patent reference 1, there is a description that an antagonist against the polypeptide can be used for the treatment for reducing the inflammatory reaction and can be administered for treating for example cardiac diseases with arteriosclerosis, inflammatory colon disease, Crohn's disease, chronic rheumatoid arthritis and pancreatitis. The patent reference 2 describes a great number of proteins comprising sequences homologous with that of RA2 and these proteins are useful for the treatment of cancer, digestive disorders, immune disorders, endocrine diseases (for example, diabetes mellitus), neurological diseases (for example, Alzheimer disease, Parkinson's disease, Creutzfeldt-Jacob disease, encephalomyelitis, menigitis, and schizophrenia) and connective tissue diseases (for example, osteoporosis and arthritis). In the patent reference 3, there is a description of a great number of genes comprising sequences homologous with the sequence of the RA2 gene and a description that on the basis of the distributions of individual expressions in tissues that these individual genes are useful for the treatment of proliferative diseases, cancer, tumor, hematopoietic diseases, immune diseases, AIDS, autoimmune diseases (for example, chronic rheumatoid arthritis), inflammation, allergy, nerological diseases (for example, Alzheimer disease), dysgnosia, schizophrenia, asthma, dermatoses (for example, psoriasis), sepsis, diabetes, artheriosclerosis, cardiovascular disorders, kidney disorders, gastrointestinal diseases, disorders related to pregnancy, endocrine disorders and infectious diseases.

A sequence homologous with the sequences of some of the polypeptides of the present invention, namely DGPP1L and DGPP1S are described in the database genpept as BC033025 (2002. 6. 24).

In the patent reference 4, the patent reference 5 and the patent reference 6, sequences homologous with DGPP2L which is one of the polypeptides of the present invention are described. Among them, in Abstract in the patent reference 4, it is described that the sequence homologous with DGPP2L is useful for the diagnosis and treatment of malignant tumor, blood diseases, HIV infection, immune diseases and various inflammations. In the patent reference 5, it is described that the sequence homologous with DGPP2L is useful for the diagnosis, treatment, preveniton and prognosis of diseases including tumor diseases such as per-proliferative diseases; that the sequence is also useful for the treatment of neurological diseases, immune diseases, muscular diseases, genital diseases, gastrointestinal diseases, lung diseases, circulatory diseases, or kidney diseases; and that the sequence is additionally useful as an agent for detecting diseases related to the abnormal expression and activity of the polypeptide and is useful for the treatment of cancer and chronic rheumatoid arthritis. In the patent reference 6, a sequence highly homologous with DGPP2L is disclosed and it is described that the sequence is useful for the detection, treatment, prevention or prognosis of per-proliferative diseases (for example, cancer), immunodeficiency diseases (for example, AIDS), autoimmune diseases (for example, arthritis), neurological diseases (for example, Alzheimer disease), metabolic disorders (for example, phenylketonuria), inflammatory diseases (for example, asthma), circulatory diseases (for example, artheriosclerosis), blood diseases (hemophilia), genital diseases (for example, infertility) and infectious diseases (for example, influenza).

In the patent reference 4, it is described that the same sequence with DGPP2S which is one of the polypeptides of the present invention, while sequences homologous with the sequence are describes in the patent reference 5 and the patent reference 6.
(Patent reference 1)
   Pamphlet of International Publication No. 00/42070
(Patent reference 2)
   Pamphlet of International Publication No. 01/77137
(Patent reference 3)
   Pamphlet of International Publication No. 01/32910
(Patent reference 4)
   Pamphlet of International Publication No. 02/26798
(Patent reference 5)
   Pamphlet of International Publication No. 01/55163
(Patent reference 6)
   Pamphlet of International Publication No. 01/55301
(Non-patent reference 1)
   *"The Journal of Experimental Medicine"*, (USA), 1991, Vol.173, p.569-574
(Non-patent reference 2)
   *"The Journal of Rheumatology"*, (Canada), 2002, Vol.29, p.875-882
(Non-patent reference 3)
   *"Current Pharmaceutical Biotechnology"*, (USA), 2000, Vol. 1, p. 217-233
(Non-patent reference 4)
   *"Nature Reviews Immunolog"*, (UK), 2002, Vol. 2, p.364-371
(Non-patent reference 5)
   *"Medicine"*, J. Axford, ed., (USA), Blackwell Science, 1996, p.3.18-3.22

### Disclosure of the Invention

As the result of various investigations, the inventors of the present invention successfully obtained six full-length gene sequences, using cDNA derived from human spleen as a template (Example 1). Five of them were novel genes. Additionally, the inventors of the present invention successfully allowed the expression of proteins encoded by the six genes in animal cell strains (Example 2). Further, the inventors of the present invention found that the expression levels of the six genes were elevated in the synovial tissues of human RA patients. Still further, the inventors of the present invention found that the expression level of each of the genes was elevated in accordance with the score of pathologic RA findings, i.e. that the severity of the inflammatory symptoms of RA is related with the elevation of the expression level of each of the genes (Example 4). Based on these findings, a method which is useful as a method for RA diagnosis by which progress of RA is detected was enabled.

From these results, the inventors of the present invention provided a novel gene, a protein encoded by the genes, expression vectors harboring the gene, a cell transformed with the expression vector or an antibody, and a detection method useful for RA diagnosis and a detecting kit therefor. Thus, the present invention has been achieved.

Specifically, the present invention relates to the following:
[1] (1) A polypeptide comprising an amino acid sequence represented by SEQ ID NO:2, SEQ ID NO:4, SEQ ID NO:6, SEQ ID NO:8 or SEQ ID NO:10 and having enhanced expression in chronic rheumatoid arthritis patients or
   (2) a polypeptide comprising an amino acid sequence represented by SEQ ID NO:2, SEQ ID NO:4, SEQ ID NO:6, SEQ ID NO:8 or SEQ ID NO:10 in which 1 to 10 amino acids are substituted, deleted and/or inserted and having enhanced expression in chronic rheumatoid arthritis patients.
[2] A polypeptide consisting of an amino acid sequence represented by SEQ ID NO:2, SEQ ID NO:4, SEQ ID NO:6, SEQ ID NO:8 or SEQ ID NO:10.
[3] A polynucleotide encoding the polypeptide according to [1] or [2].
[4] An expression vector comprising the polynucleotide according [3].
[5] A cell transformed with the expression vector according to [4].
[6] A antibody which binds to a polypeptide comprising an amino acid sequence represented by SEQ ID NO:4 and having enhanced expression in chronic rheumatoid arthritis patients, a polypeptide comprising an amino acid sequence represented by SEQ ID NO:4 in which 1 to 10 amino acids are substituted, deleted and/or inserted and having enhanced expression in chronic rheumatoid arthritis patients or a polypeptide consisting of an amino acid sequence represented by SEQ ID NO:4.
[7] A polynucleotide which specifically hybridizes to a polynucleotide represented by SEQ ID NO:1, SEQ ID NO:3, SEQ ID NO:5, SEQ ID NO:7 or SEQ ID NO:9 and has at least 15 nucleotides.
[8] A method for detecting rheumatoid arthritis comprising
   1) a step of assaying the expression level of a gene in a synovial biopsy sample of a test subject, said gene comprising
      i) the nucleotide sequence according to [3] or
      ii) a polynucleotide sequence encoding a polypeptide comprising an amino acid sequence represented by SEQ ID NO:12 and having enhanced expression in chronic rheumatoid arthritis patients, a polypeptide comprising an amino acid sequence represented by SEQ ID NO:12 in which 1 to 10 amino acids are substituted, deleted and/or inserted and having enhanced expression in chronic rheumatoid arthritis patients, or a polypeptide consisting of an amino acid sequence represented by SEQ ID NO:12; and
   (2) a step of comparing the expression level of the gene with the expression levels thereof in a synovial biopsy sample in a normal subject or a patient with non-chronic rheumatoid arthritis.
[9] A kit for detecting chronic rheumatoid arthritis comprising forward and reverse primers designed for enabling specific amplification of a gene represented by
   1) the polynucleotide according to [3] or
   2) a polynucleotide encoding a polypeptide comprising an amino acid sequence represented by SEQ ID NO:12 and having enhanced expression in chronic rheumatoid arthritis patients, a polypeptide comprising an amino acid sequence represented by SEQ ID NO:12 in which 1 to 10 amino acids are substituted, deleted and/or inserted and having enhanced expression in chronic rheumatoid arthritis patients, or a polypeptide consisting of an amino acid sequence represented by SEQ ID NO:12.

Although the same sequence with RA2 which is one of the polypeptides of the present invention has not yet been known, a homologous sequence is registered as AK094461 (2002. 7. 15) on the database genpept and is also described in the patent reference 1, the patent reference 2 and the patent reference 3. The patent reference 1, the patent reference 2 and the patent reference 3 cites a great number of diseases which the polypeptide relates to and includes chronic rheumatoid arthritis and arthritis among them. However, in the patent reference 1, it is described that by only the reason that the molecule is expressed in a lymph node, a spleen, a thymus, a btestis, a small intestine, a human arterial endothelial cell, a smooth muscle, a kidney, a mast cell, an eosinophilic leukocyte, a tonsil, a pancreas, a large intestine, a peripheral blood lymphocyte, a stomach, a tachea, T cells including CD4⁺ and CD8⁺ and bone marrow, the molecule is described as a cytokine and is described that antagonists against the molecule are useful for inflammatory diseases including RA. In the patent reference 2, any example which relates to the molecule is not described. In the patent reference 3, although plural molecules are described, the examples are simply described in present form. Additionally, the patent references 1 through 3 never include any description about the presence or absence of the expression of the molecule in a synovial membrane or about the expression level thereof in RA patients. Accordingly, the fact that RA2 is useful for RA diagnosis was found by the inventors of the present invention for the first time. Further, the method for detecting RA based on the finding is present invention achieved by the inventors of the present invention for the first time.

The same or highly homologous sequence with RA3 which is one of the polypeptides of the present invention has never been known. Thus, RA3 is a polypeptide found and obtained by the inventors of the present invention for the first time. The elevation of the RA expression level according to the severity of the inflammatory symptoms of RA was found by the inventors of the present invention for the first time.

The same sequences with DGPP1L and DGPP1S which are one of the polypeptides of the present invention have never been known. Although a sequence homologous with them is registered as BC033025 on the database genpept (2002. 6. 24), the function is unknown. Further, it was found by the inventors of the present invention for the first time that DGPP1L and DGPP1S are useful for detecting RA.

The same sequence with DGPP2L which is one of the polypeptides of the present invention has not yet been known. However, homologous sequences are described in the patent reference 4, patent reference 5, and patent reference 6. Among them, in Abstract in the patent reference 4, it is described that the sequence homologous with DGPP2L is useful for the diagnosis and treatment of malignant tumor, blood diseases, HIV infection, immune diseases and various inflammations. In the patent reference 5, it is disclosed that a great number of sequences including sequences homologous with DGPP2L, and these polypeptides are useful for the diagnosis, treatment, prevention and prognosis of diseases including tumor diseases such as per-proliferative diseases; that the sequences are also useful for the treatment of nerological diseases, immune diseases, muscular diseases, genital diseases, gastrointestinal diseases, lung diseases, circulatory diseases, or kidney diseases; and that the sequences are useful as an agent for detecting diseases involved in the abnormal expression and activity of the polypeptide and are useful for the treatment of cancer and chronic rheumatoid arthritis. However, in the reference, there is no disclosure about which sequence among these many sequences can be used for which use and about examples carried out actually.

In the patent reference 6, it is disclosed that a great number of sequences including sequences highly homologous with DGPP2L and it is described that the sequences are useful for the detection, treatment, prevention or prognosis such as per-proliferative diseases (for example, cancer), immunodeficiency diseases (for example, AIDS), autoimmune diseases (for example, arthritis), neurological diseases (for example, Alzheimer disease), metabolic disorder (for example, phenylketonuria), inflammatory diseases (for example, asthma), circulatory diseases (for example, atheroma arteriosclerosis), blood diseases (hemophilia), genital diseases (for example, infertility) and infectious diseases (for example, influenza). However, in the patent reference 6, there is neither any specific description about examples nor specific supporting evidence for these uses. Further, in the patent references 5 and 6, there is not any description of the presence or absence of the expression of a DGPP2L analogous molecule in a synovial membrane or about the expression level thereof in RA patients. Accordingly, it is the fact found by the inventors of the present invention for the first time that DGPP2L is useful for RA diagnosis. Further, the method for detecting RA based on the finding is an invention achieved by the inventors of the present invention for the first time.

In the patent reference 4 described above, the same sequence with DGPP2S which is one of the polypeptide of the present invention is described, while sequences homologous with the sequence are described in the patent reference 5 and the patent reference 6. As described above, however, there is not any description in the patent reference 5 and the patent reference 6 about the presence or absence of the expression of the molecule in a synovial membrane or about the expression level thereof in RA patients. Accordingly, it was found for the first time by the inventors of the present invention that DGPP2L is useful for RA diagnosis. Further, the method for detecting RA based on the finding is an invention achieved for the first time by the inventors of the present invention.

### Brief Description of the Drawings

Fig. 1 shows the expressions of the individual novel proteins.
Fig. 2 shows the expression levels of the individual gene in RA synovial fibroblast-like cells in comparison with these in OA fibroblast-like cells. The vertical axis in the figure represents relative expression level.

### Best Mode for Carrying out the Invention

The present invention is now described in detail hereinbelow.

The polypeptides of the present invention include:
(1) a polypeptide consisting of an amino acid sequence represented by SEQ ID NO:2, SEQ ID NO:4, SEQ ID NO:6, SEQ ID NO:8 or SEQ ID NO:10;
(2) a polypeptide comprising an amino acid sequence represented by SEQ ID NO:2, SEQ ID NO:4, SEQ ID NO:6, SEQ ID NO:8 or SEQ ID NO:10 and having enhanced expression specific to RA patients or a polypeptide comprising an amino acid sequence represented by SEQ ID NO:2, SEQ ID NO:4, SEQ ID NO:6, SEQ ID NO:8 or SEQ ID NO:10 in which 1 to 10 amino acids in an amino acid and having enhanced expression specific to RA patients; (called functionally equivalent variant hereinafter); and
(3) a polypeptide comprising an amino acid sequence with 90 % or more homology with an amino acid sequence represented by SEQ ID NO:2, SEQ ID NO:4, SEQ ID NO:6, SEQ ID NO:8 or SEQ ID NO:10 or SEQ ID NO:12 and having enhanced expression specific to RA patients (called homologous polypeptide hereinbelow).

The "functionally equivalent variant of the present invention" includes "a polypeptide comprising an amino acid sequence represented by SEQ ID NO:2, SEQ ID NO:4, SEQ ID NO:6, SEQ ID NO:8 or SEQ ID NO:10 and having enhanced expression specific to RA patients" or "a polypeptide comprising an amino acid sequence represented by SEQ ID NO:2, SEQ ID NO:4, SEQ ID NO:6, SEQ ID NO:8 or SEQ ID NO:10 in which 1 to 10, preferably 1 to 7, more preferably 1 to 5 amino acids are substituted, deleted and/or inserted and having enhanced expression specific to RA patients".

Although the "homologous polypeptide of the present invention" is not limited as far as "a polypeptide comprising an amino acid sequence with 90 % or more homology with an amino acid sequence represented by SEQ ID NO:2, SEQ ID NO:4, SEQ ID NO:6, SEQ ID NO:8 or SEQ ID NO:10 and having enhanced expression specific to RA patients". Preferably, the homologous polypeptide of the present invention is a polypeptide comprising an amino acid sequence with preferably 95% or more homology, more preferably 98% or more homology.

In this specification, the "homology" means the value of Identities obtained by using the bl2seq program (Tatiana A. Tatsusova, Thomas L. Madden, *FEMS Microbiol. Lett.,* 174, 247-250, 1999) in the BLAST PACKAGE [sgi32 bit edition, Version 2.0.12; available from National Center for Biotechnology Information (NCBI)]. As the parameters, herein, pair wise alignment parameters are individually used, including "balstp" as "program name", "0" as "Gap insertion Cost value", "0" as "Gap extension Cost value", and "BLOSUM62" as "Matrix".

The phrase "enhanced expression specific to RA patients" means expression enhanced 2-fold or more in the synovial tissue or synovial fibroblast-like cell of RA patients, compared with non-RA patients.

The polypeptides of the present invention have been described so far. The polypeptide comprising an amino acid sequence represented by SEQ ID NO:2, SEQ ID NO:4, SEQ ID NO:6, SEQ ID NO:8 or SEQ ID NO:10, the functionally equivalent variant of the present invention and the homologous polypeptide of the present invention are generally called as "the polypeptide of the present invention" hereinafter. Further, "the polypeptide of the present invention", a polypeptide comprising an amino acid sequence represented by SEQ ID NO:12 and having enhanced expression specific to RA patients, a polypeptide comprising an amino acid sequence represented by SEQ ID NO:12 in which 1 to 10, preferably 1 to 7, more preferably 1 to 5 amino acids are substituted, deleted and/or inserted and having enhanced expression specific to RA patients, or a polypeptide consisting of an amino acid sequence represented by SEQ ID NO:12 is generally called as "the polypeptide for the detection method of the present invention" hereinafter. In "the polypeptide for the detection method of the present invention", a protein which is the polypeptide consisting of an amino acid sequence represented by SEQ ID NO:2 is called as "RA2 protein"; a protein which is the polypeptide consisting of an amino acid sequence represented by SEQ ID NO:4 is called as "RA3 protein"; a protein which is the polypeptide consisting of an amino acid sequence represented by SEQ ID NO:6 is called as "DGPP1L protein"; a protein which is the polypeptide consisting of an amino acid sequence represented by SEQ ID NO:8 is called as "DGPP1S protein"; a protein which is the polypeptide consisting of an amino acid sequence represented by SEQ ID NO:10 is called as "DGPP2L protein"; and a protein which is the polypeptide consisting of an amino acid sequence represented by SEQ ID NO:12 is called as "DGPP2S protein".

The polypeptide of the present invention is preferably "a polypeptide consisting of an amino acid sequence represented by SEQ ID NO:2, SEQ ID NO:4, SEQ ID NO:6, SEQ ID NO:8 or SEQ ID NO:10", " a polypeptide comprising an amino acid sequence represented by SEQ ID NO:2, SEQ ID NO:4, SEQ ID NO:6, SEQ ID NO:8 or SEQ ID NO:10 and involving enhanced expression specific to RA patients or a polypeptide comprising an amino acid sequence represented by SEQ ID NO:2, SEQ ID NO:4, SEQ ID NO:6, SEQ ID NO:8 or SEQ ID NO:10 in which 1 to 10, preferably 1 to 7, more preferably 1 to 5 amino acids are substitutes, deleted and/or inserted and having enhanced expression specific to RA patients", or "a polypeptide comprising an amino acid sequence with 90% or more (preferably 95% or more, more preferably 98% or more) homology with an amino acid sequence represented by SEQ ID NO:2, SEQ ID NO:4, SEQ ID NO:6, SEQ ID NO:8 or SEQ ID NO:10 and having enhanced expression specific to RA patients". The polypeptide of the present invention is more preferably "a polypeptide consisting of an amino acid sequence represented by SEQ ID NO:2, SEQ ID NO:4, SEQ ID NO:6, SEQ ID NO:8 or SEQ ID NO:10".

Additionally, the polynucleotide consisting of a nucleotide sequence encoding the RA2 protein of the present invention may be any of a nucleotide sequence encoding the RA2 protein represented by the amino acid sequence of SEQ ID NO:2, a functionally equivalent variant thereof or a homologous polypeptide thereof. The polynucleotide is preferably a polynucleotide consisting of a nucleotide sequence encoding the amino acid sequence of SEQ ID NO:2 and is more preferably the nucleotide sequence of SEQ ID NO:1.

The polynucleotide consisting of a nucleotide sequence encoding the RA3 protein of the present invention may be any of a nucleotide sequence encoding the RA3 protein represented by the amino acid sequence of SEQ ID NO:4, a functionally equivalent variant thereof or a homologous polypeptide thereof. The polynucleotide is preferably a polynucleotide consisting of a nucleotide sequence encoding the amino acid sequence of SEQ ID NO:4 and is more preferably the nucleotide sequence of SEQ ID NO:3.

The polynucleotide consisting of a nucleotide sequence encoding the DGPP1L protein of the present invention may be any of a nucleotide sequence encoding the DGPP1L protein represented by the amino acid sequence of SEQ ID NO:6, a functionally equivalent variant thereof or a homologous polypeptide thereof. The polynucleotide is preferably a polynucleotide consisting of a nucleotide sequence encoding the amino acid sequence of SEQ ID NO:6 and is more preferably the nucleotide sequence of SEQ ID NO:5.

The polynucleotide consisting of a nucleotide sequence encoding the DGPP1S protein of the present invention may be any of a nucleotide sequence encoding the DGPP1S protein represented by the amino acid sequence of SEQ ID NO:8, a functionally equivalent variant thereof or a homologous polypeptide thereof. The polynucleotide is preferably a polynucleotide consisting of a nucleotide sequence encoding the amino acid sequence of SEQ ID NO:8 and is more preferably the nucleotide sequence of SEQ ID NO:7.

The polynucleotide consisting of a nucleotide sequence encoding the DGPP2L protein of the present invention may be any of a nucleotide sequence encoding the DGPP2L protein represented by the amino acid sequence of SEQ ID NO:10, a functionally equivalent variant thereof or a homologous polypeptide thereof. The polynucleotide is preferably a polynucleotide consisting of a nucleotide sequence encoding the amino acid sequence of SEQ ID NO:10 and is more preferably the nucleotide sequence of SEQ ID NO:9.

The polynucleotide consisting of a nucleotide sequence encoding the DGPP2S protein of the present invention may be any of a nucleotide sequence encoding the DGPP2S protein represented by the amino acid sequence of SEQ ID NO:12, a functionally equivalent variant thereof or a homologous polypeptide thereof. The polynucleotide is preferably a polynucleotide consisting of a nucleotide sequence encoding the amino acid sequence of SEQ ID NO:12 and is more preferably the nucleotide sequence of SEQ ID NO:11.

The method for producing a polynucleotide (including the polynucleotide of the present invention) encoding the polypeptide for the detection method of the present invention and a polynucleotide specifically hybridizing with the polynucleotide of the present invention is not particularly limited and for example includes (1) the PCR method; (2) a method using routine genetic engineering technique (in other words, a method including selecting a transformant strain containing desired amino acids from transformant strains obtained by transformation with cDNA library); or (3) a chemical synthesis method. The production methods can be individually carried out as described in WO 01/34785. Herein, the "novel protein of the present invention" in the specification of the patent application should be read as the polypeptide for the detection method of the present invention (namely, the RA2 protein, the RA3 protein, the DGPP1L protein, the DGPP1S protein, the DGPP2L protein or the DGPP2S protein), while the "gene of the present invention" therein should be read as the gene encoding the polypeptide for detection in accordance with the present invention (namely, the RA2 gene, the RA3 gene, the DGPP1L gene, the DGPP1S gene, the DGPP2L gene or the DGPP2S gene). Hereinafter, the polynucleotide of the present invention is described as an example. However, the polynucleotide encoding the polypeptide for the detection method of the present invention (more specifically, a polypeptide comprising an amino acid sequence represented by SEQ ID NO:12 and having enhanced expression specific to chronic rheumatoid arthritis patients, a polypeptide comprising an amino acid sequence represented by SEQ ID NO:12 in which 1 to 10, preferably 1 to 7, more preferably 1 to 5 amino acids are substituted, deleted, and/or inserted and having enhanced expression specific to chronic rheumatoid arthritis patients, or a polypeptide consisting of an amino acid sequence represented by SEQ ID NO.12 can also be produced in the same manner.

By the method using PCR, for example, the polynucleotide of the present invention can be produced by procedures described in "Mode for Carrying out the Invention", 1) Method for producing protein gene, a) First production method in the patent reference described above. A mRNA is extracted from cells or tissues with an ability of producing the protein of the present invention, for example from human RA patient-derived synovial membrane. Then, the mRNA is subjected to reverse-transcriptase reaction in the presence of random primer or oligo dT primer, to synthetically prepare a first cDNA chain. Using the obtained first cDNA chain and two primer types directed for a partial region of the intended gene, the cDNA is treated by polymerase chain reaction (PCR), to obtain the polynucleotide of the present invention as a whole or as a part. More specifically, the polynucleotide of the present invention can be produced for example by the method described in Example 1.

In the method using routine genetic engineering technique, for example, the polynucleotide of the present invention can be produced by the procedures described in "Mode for Carrying out the Invention", 1) Method for producing protein gene, a) Second production method in the patent reference described above.

By the method using chemical synthesis, the polynucleotide of the present invention can be produced for example by the methods described in, "the Mode for Carrying out the Invention", 1) Method for producing protein gene, c) Third production method and d) Fourth production method in the patent reference described above. More specifically, the polynucleotide of the present invention may be produced by binding together nucleotide fragments produced by chemical synthetic method. Additionally, each polynucleotide (oligonucleotide) may also be synthetically produced, using a DNA synthesizer (for example, Oligo 1000M DNA Synthesizer (Beckman) or 394 DNA/RNA Synthesizer (Applied Biosystems)).

The methods for producing expression vector, host cell and protein in the present invention can be produced by methods described in, "Mode for carrying out the Invention", 2) Methods for producing the vector of the present invention, the host cell of the present invention and the recombinant protein of the present invention in the patent reference described above. The isolated polynucleotide of the present invention is integrated into an appropriate vector DNA again, to thereby transform eukaryotic or prokaryotic host cells. By additionally introducing an appropriate promoter and a sequence which relates to the phenotypic expression, the polynucleotide can be expressed in individual host cells.

The expression vector of the present invention is not particularly limited as far as it comprises the polynucleotide of the present invention, and includes such as an expression vector obtained by inserting the polynucleotide of the present invention into known expression vectors appropriately selected, depending on the host cell used.

Further, the cell of the present invention is not particularly limited as far as it is transfected with the expression vector of the present invention described above and it comprises the polynucleotide of the present invention, and includes for example cells in which the polynucleotide of the present invention is integrated in the chromosome of the host cells, or cells containing the polynucleotide of the present invention in the form of an expression vector comprising the polynucleotide. Additionally, the cell may be a cell which expresses the polypeptide of the present invention or a cell which does not expresses the polypeptide of the present invention. The cell of the present invention can be obtained by transfecting a desired host cell with the expression vector of the present invention. More specifically, for example, as described in Example 1 and Example 2, by integrating the polynucleotide of the present invention in an expression vector pcDNA3.1 for mammalian cells, an expression vector for a desired protein can be obtained, and then, by incorporating the expression vector in a human fetal kidney-derived 293T cell with the use of a commercially available transfection reagent Lipofectamine 2000, the transformant cell of the present invention can be produced.

The desired transformant cell obtained above can be cultured by routine methods. Through the culturing, the protein of the present invention is produced. As the medium for use in the culture, various medium routinely used can be selected appropriately in accordance with a selected host cell. For the 293T cell, for example, the Dulbecco's modified Eagle's minimum essential culture medium (DMEM) supplemented for example with a serum component such as fetal bovine serum (FBS) and additionally supplemented with G418 may be used.

The protein of the present invention produced in the transformant cell as described above can be separated and purified by various known separation procedures and methods using the physical properties and biochemical properties of the protein and the like.

The protein of the present invention is fused in-frame with a marker sequence and is then expressed, to enable the identification and purification of the protein and the like. As the marker sequence, for example, FLAG epitope, hexa-histidine tag, hemagglutinin tag, and mye epitope are known. By additionally inserting amino acid sequences specifically recognized by proteases, such as enterokinase, Factor Xa and thrombin in between the marker sequence and the novel protein, the marker sequence part can be cleaved and removed with these proteases.

The polynucleotide of the present invention per se or a part thereof can be used as a hybridization probe in the detection method of RA as described below and is therefore useful for detection of RA. Additionally, the polypeptide of the present invention can be used for producing an antibody which specifically recognizes the polypeptide of the present invention or as a control for detecting and assaying the expression level.

### <Method for producing the antibody of the invention>

Although the method for producing the antibody of the present invention is not particularly limited, it can be carried out in the same manner as described in the "Mode for Carrying out the Invention", <Antibody binding to the polypeptide of the invention> in the patent reference described above. The antibody of the present invention, for example the polyclonal antibody or monoclonal antibody, can be obtained by direct administration of a polypeptide comprising an amino acid sequence represented by SEQ ID NO:4 and having enhanced expression specific to chronic rheumatoid arthritis patients, a polypeptide comprising an amino acid sequence represented by SEQ ID NO:4 in which 1 to several amino acids are substituted, deleted, and/or inserted, and having enhanced expression specific to chronic rheumatoid arthritis patients, or the whole or a part of a polypeptide consisting of an amino acid sequence represented by SEQ ID NO:4 to various animals. Additionally, the antibody may be obtained by a DNA vaccination method using a plasmid in which the gene encoding the polypeptide is introduced (Raz, E. *et al., Proc. Natl. Acad. Sci. USA*, **91,** 9519-9523, 1994; Donnelly, J. J. *et al.,* J. Infect. Dis., **173,** 314-320, 1996).

Such polyclonal antibody can be produced from the serum or egg of a sensitized animal, for example sensitized rabbit, rat, goat or chicken, which is prepared by emulsifying the polypeptide or a part thereof in an appropriate adjuvant such as Freund's complete adjuvant and immunizing an animal intraperitoneally, subcutaneously or intravenously. The polyclonal antibody thus produced can be separated and purified by routine methods for protein isolation and purification. The routine methods for protein isolation and purification include for example centrifugation, dialysis, salting out with ammonium sulfate and chromatography for example with DEAE-cellulose, hydroxyapatite, and Protein A agarose.

The monoclonal antibody can be produced easily by a person skilled in the art according to the cell fusion method of Kohler and Milstein (Kohler, G. and Milstein, C., *Nature,* **256,** 495-497, 1975).

### <Polynucleotide specifically hybridizing to the polynucleotide of the invention>

A polynucleotide specifically hybridizing with a polynucleotide represented by SEQ ID NO:1, SEQ ID NO:3, SEQ ID NO:5, SEQ ID NO:7 or SEQ ID NO:9 and having at least 15 nucleotides is also included in the present invention. The phrase "specifically hybridizing" with the polynucleotide of the present invention means hybridizing with the polynucleotide of the present invention but never hybridizing with other polynucleotides under general hybridization condition, preferably under strict condition. The strict condition broadly means conditions for hybridization, such as about "5 x SSPE, 5 x Denhardt's solution, 0.5% SDS, 40% formamide, 200 µg/ml salmon sperm DNA, and 37°C overnight". More strict condition mean condition such as about "5 x SSPE, 5 x Denhardt's solution, 0.5% SDS, 50% formamide, 200 µg/ml salmon sperm DNA, and 42 °C overnight". As washing condition, lax condition is the one such as about "5 × SSC, 1% SDS and 42°C", general condition is such as about "0.5 × SSC, 0.1% SDS and 42°C", and more strict condition is such as about "0.2 × SSC, 0.1% SDS and 65°C". Such polynucleotide can be used as probe for the detection and isolation of the polynucleotide of the present invention or as primer for the amplification of the polynucleotide of the present invention. In case that the polynucleotide is to be used as primer, generally, the polynucleotide is of a chain length of 15 bp to 100 bp, preferably 15 bp to 40 bp. Preferable nucleotide sequences as such primer include 1) primers represented by the nucleotide sequences of SEQ ID NOS: 25 and 26, 2) primers represented by the nucleotide sequences of SEQ ID NOS: 27 and 28, 3) primers represented by the nucleotide sequences of SEQ ID NOS: 29 and 30, and 4) primers represented by the nucleotide sequences of SEQ ID NOS: 31 and 32. In case that the polynucleotide is to be used as probe, DNA comprising at least a part of the sequence of the polynucleotide or the whole (or a complimentary sequence) of the present invention and having a chain length of at least 15 bp is used.

The probe and the primer in accordance with the present invention can be used for assaying the expression level of the gene of the present invention for detection for RA diagnosis.

In accordance with the present invention, an array of oligonucleotide probes comprising the nucleotide sequence of the polynucleotide of the present invention or a fragment thereof can be constructed. The array technique is known and used for analyzing gene expression (Chee, M. *et al.* (1996) *Science,* **274,** 610-613).

### <RA detection method/Kit for RA detection>

Since the genes which has an elevated expression levels in the synovial tissues of human RA patients was found using comparison with the synovial tissues of human osteoarthritis patients (OA), the RA disease can be detected using the expression levels thereof. So as to identify RA-specific phenomena, OA with a disorder in the synovial tissue was used as a control. Specifically, a mode including the following steps is exemplified. In other words, the mode includes
1) a step of assaying the expression level of a gene comprising a polynucleotide sequence encoding the polypeptide for the detection method of the present invention in a synovial biopsy sample of a test subject and
2) a step of comparing the expression level of the gene with the expression levels thereof in synovial biopsy samples from normal subjects or patients with non-chronic rheumatoid arthritis.

The gene expression level for the detection method of RA in the present invention includes the gene transcription into mRNA and its translation into protein. Thus, the detection method of RA in accordance with the present invention is carried out by comparison with the expression level of mRNA corresponding to the gene of the present invention or the expression level of the protein encoded by the gene.

The method for assaying the expression level of a gene comprising a polynucleotide sequence encoding the polypeptide for the detection method of the present invention in the step (1) can be carried out according to known gene analysis methods. For example, hybridization technique using nucleic acid which hybridizes with the gene of the present invention as probe, or gene amplification technique using DNA which hybridizes with a polynucleotide encoding the polypeptide for the detection method of the present invention as probe can be utilized. Specifically, nucleic acid derived from synovial cells from a test subject, for example mRNA can be used for the assay. The mRNA amount can be assayed by the gene amplification method using a primer designed so as to specifically amplify the gene sequence of the present invention. The gene amplification method is not particularly limited and for example, PCR and nucleic acid amplification method using RNA polymerase can be used with no specific limitation. The primer for use in the detection method of RA in the present invention or the primer contained in the kit for RA detection in the present invention are not particularly limited as far as it can specifically amplify the gene sequence encoding the polypeptide for the detection method of the present invention. Such primer can be designed on the basis of the nucleotide sequence of the gene of the present invention. For the PCR amplification monitor method, is enabled by using for example a primer design software Primer Express (PE Biosystems). Additionally, a polynucleotide specifically hybridizing with a polynucleotide encoding the polypeptide for the detection method in the present invention can also be used as such primer.

RA detection using hybridization technique can be carried out, using for example Northern hybridization, dot blot method and DNA microarray method. Additionally, gene amplification technique such as RT-PCR may also be used. By RT-PCR, PCR amplification monitor (real-time PCR) method (Genome Res., **6**(10), 986, 1996) is used during the course of gene amplification , to thereby make a more quantitative assay of the expression of the gene of the present invention. As the PCR amplification monitor method, for example ABI PRISM 7900 (PE Biosystems) can be used. Real-time PCR is a known method, and apparatuses and kits therefor are commercially available. Using them, the method can be carried out in a simple manner. More specifically, the method can be carried out by the method described in Example 4.

At the step (1), further, as the method for assaying the expression level of the gene comprising a polynucleotide sequence encoding the polypeptide for the detection method of the present invention, a method for detecting a protein expressed by the gene can be used. As such detection method, for example, Western blotting, immunoprecipitation method and ELISA can be used, using an antibody binding to the protein, preferably an antibody specifically binding to the protein with a cell extract solution from the synovial cell obtained from a test subject.

At the step (2), comparative method is not particularly limited as far as comparing the expression level obtained at the step (1) with the expression levels in normal subjects or non-RA patients. For example, such comparison can be carried out by the method described in Example 4.

The kit for RA detection in the present invention includes at least a forward primer and a reverse primer designed for the specific amplification of a polynucleotide encoding the polypeptide for the detection method in the present invention.

A pair of the forward and reverse primers includes for example polynucleotides specifically hybridizing with a polynucleotide encoding the polypeptide for the detection method in the present invention. Other reagents which may be contained in the kit for RA detection in the present invention include for example reagents required for PCR (for example, Taq polymerase, nucleotide substrates, buffers, etc.).

### Examples

Although the present invention is now described in more detail in the following Examples, the present invention is never limited by the Examples. Unless otherwise stated, gene manipulation experimental manuals for known methods (for example, Sambrook, J. *et al., "Molecular Cloning-A Testing Manual",* Cold Spring Harbor Laboratory, NY, 1989) and instructions attached to reagents and the like were followed.

### Example 1

### Cloning of full-length open reading frame (ORF) and construction of plasmid expressing protein

Using a primer set shown in Table 1 (SEQ ID NO:13 and SEQ ID NO:14, SEQ ID NO:15 and SEQ ID NO:16, SEQ ID NO:17 and SEQ ID NO:18, SEQ ID NO:19 and SEQ ID NO:18, SEQ ID NO:20 and SEQ ID NO:21, or SEQ ID NO:22 and SEQ ID NO:21), human spleen-derived cDNA (Human spleen 5'-stretch plus cDNA; Invitrogen) and DNA polymerase (Pyrobest (trade mark) DNA polymerase; Takara Shuzo Co., Ltd.), PCR was carried out at 94°C for 2 minutes and 20 times of cycle consisting of 98°C for 10 seconds, 60°C for 30 seconds and 72°C for 1 minute and 30 seconds, and followed by at 72°C for 3 minutes. The PCR product was treated with phenol and chloroform, precipitated with ethanol and dissolved in distilled water. In the obtained DNAs, DNA amplified with the primer set of SEQ ID NO:13 and SEQ ID NO: 14 was cleaved doubly with *BamH*I and *Xho*I, while other DNAs were doubly cleaved with *Hin*dIII and *Xho*I. The obtained products were inserted at the *BamH*I*-Xho*I site or at the *Hind*III*-Xho*I site of the expression vector pcDNA3.1-CFL. The each obtained expression plasmid was designated as CFL-RA2, CFL-RA3, CFL-DGPP1L, CFL-DGPP1S, CFL-DGPP2L, and CDL-DFPP2S. A pcDNA3.1-CFL is a plasmid prepared by inserting a double-stranded oligoDNA consisting of SEQ ID No: 23 and SEQ ID NO:24 in the *Xho*I*-Xba*I site of pcDNA3.1(+) (Invitrogen). The plasmid gives the FLAG tag to intended protein, which is then expressed. The plasmids were sequenced by dideoxy terminator method, using AB13700 DNA sequencer (Applied Biosystems), to obtain a sequence represented by SEQ ID NO:1, SEQ ID NO:3, SEQ ID NO:5, SEQ ID NO:7, SEQ ID NO:9 or SEQ ID NO:11. The full-length ORF sequence of each of the sequences was determined. The genes were individually designated as RA2, RA3, DGPP1L, DGPP1S, DGPP2L and DGPP2S. Putative amino acid sequences were shown in SEQ ID NO:2, SEQ ID NO:4, SEQ ID NO:6, SEQ ID NO:8, SEQ ID NO:10 and SEQ ID NO:12. RA2 ORF represented by SEQ ID NO:2 encoded a novel protein consisting of 357 amino acids; RA3 ORF represented by SEQ ID NO:4 encoded a novel protein consisting of 101 amino acids; DGPP1L ORF represented by SEQ ID NO:6 encoded a novel protein consisting of 313 amino acids; DGPP1S ORF represented by SEQ ID NO:8 encoded a novel protein consisting of 264 amino acids; DGPP2L ORF represented by SEQ ID NO:10 encoded a novel protein consisting of 291 amino acids; and DGPP2S ORF represented by SEQ ID NO:12 encoded a novel protein consisting of 271 amino acids.

**Table 1**

| | Forward primer (sequence with recognition sequence of a restriction enzyme *Bam*HI or *Hin*dIII at 5' end) | Reverse primer (sequence with recognition sequence of a restriction enzyme *Xho*I at 5' end) |
|---|---|---|
| CFL-RA2 | SEQ ID NO:13 | SEQ ID NO:14 |
| CFL-RA3 | SEQ ID NO:15 | SEQ ID NO:16 |
| CFL-DGPP1L | SEQ ID NO:17 | SEQ ID NO:18 |
| CFL-DGPP1S | SEQ ID NO:19 | SEQ ID NO:18 |
| CFL-DGPP2L | SEQ ID NO:20 | SEQ ID NO:21 |
| CDL-DFPP2S | SEQ ID NO:22 | SEQ ID NO:2 |
| FLAG | SEQ ID NO:23 | SEQ ID NO:24 |

### Example 2 Expression in animal cell strain

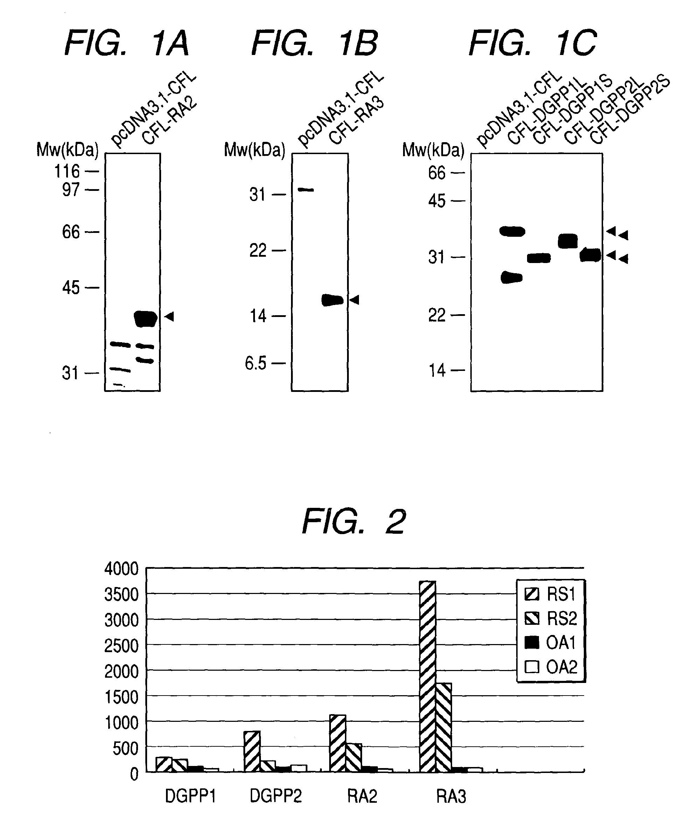

The six expression plasmids prepared in Example 1 were individually introduced in 293T cell using a transfection reagent (Lipofectamine; Gibco) according to the attached instruction. The culture medium was replaced with a serum-free culture medium 12 to 16 hours after the introduction of the plasmids, followed by additional continuous culturing for another 48 to 60 hours to collect cells A solution consisting of 50 mM Tris, pH 7.5, 0.25 M NaCl, 10% glycerol, 1% Triton X-100, 1 mM EDTA, 1 mM EGTA and 1 mM PMSF (Sigma) was added to the obtained cells to dissolve the cells, followed by centrifugation at 15,000 rpm for 15 minutes with an Eppendorf tube centrifuge machine (Tomy Digital Biology Co., Ltd.) to obtain the supernatant. The presence of an intended protein in the lysate of the introduced cell was confirmed by Western blotting using an antibody (mouse anti-FLAG monoclonal antibody M2; Sigma) against the FLAG tag added at the C terminus. In other words, the lysate described above was electrophoresed (under the reducing condition) on SDS/4 % to 20 % acrylamide gel (Daiichi Pure Chemicals Co., Ltd.) and then transferred onto PVDF membrane (Millipore), using a blotting apparatus. After Block Ace (DAINIPPON PHARMACEUTICAL CO., LTD.) was added to the PVDF membrane for blocking after the transfer, a biotinylated mouse anti-FLAG monoclonal antibody and horseradish peroxidase-labeled streptoavidin (Amersham Pharmacia) were sequentially added for reaction. After reaction, the expression of the intended protein was confirmed using ECL Western blotting detection system (Amersham Pharmacia). In the lysate of the cell introduced with each of the expression plasmids of RA2, DGPP1L, DGPP1S, DGPP2L and DGPP2S, a band located around a position of each putative molecular weight (RA2: 40.1 kDa; DGPP1L: 34.3 kDa; DGPP1S: 29.5 kDa; DGPP2L: 32.3 kDa and DGPP2S: 30.4 kDa) was detected, and it can be known that the intended proteins were expressed in the each introduced cells (Fig. 1). Since RA3 has a high content of proline residue, the mobility by electrophoresis is very low and RA3 was observed at a position of a larger molecular weight than the putative molecular weight (11.0 kDa) (Fig. 1).

### Example 3

### Obtaining synovial tissue sample and synovial fibroblast-like cell sample

Synovial tissues and synovial fibroblast-like cells were recovered via synovial membrane biopsy from human RA patients and human OA patients. Synovial cells were prepared according to the reference of Harigai M, *et al. (J Rheumatol*. 1999 May; **26**(5): 1035-43) while synovial fibroblast-like cells were prepared according to the reference of Zhang HG, *et al. (Arthritis Rheum*. 2000 May; **43**(5): 1094-105). G1 and G6 were samples of synovial tissues in mixture from two human RA patients. The score of various pathological findings was as follows: G1:1.5 and G6:0 in the modification of surface layer into multi layer; G1:2.5 and G6:0 in the formation of lymph follicle; G1:2.0 and G6:0.5 angiogenesis. G7 is a sample of synovial tissues in mixture from two human OA patients. The score of pathological findings was based on Harigai M., *et al*., *Clin Immunol Immunopathol*. 1993 Oct; **69**(1): 83-91 and reflects the inflammation level of synovial tissue. G1 with a high score of pathological findings is a sample with a larger inflammation level than that of G6.

R1 and R2 are synovial fibroblast-like cell samples from individual human RA patients. The scores of pathological findings are as follows: RS1:2 and RS2:0 in the modification of surface layer into multi layer; R1:2 and RS2:0 in the formation of lymph follicle; and RS1:3 and RS2:2 concerning angiogenesis. In other words, when comparing RS1 with RS2, RS1 is a sample with a larger inflammation level than that of RS2. OA1 and OA2 are synovial fibroblast-like cell samples from one human OA patient.

### Example 4

### Elevation of each gene expression in synovial tissues and synovial fibroblast-like cells

Real-time PCR with ABI PRISM 7900HT Sequence Detection System (PE Biosystems) (referred to as Prism 7900 hereinafter) was carried out using a PCR quantitative assay reagent kit SYBR Green PCR Master Mix (PE Biosystems) according to the instruction attached to the kit, to quantitatively assay the expression level of each gene in synovial tissues and synovial fibroblast-like cells. The detail is now described below. The primers for use in assaying with ABI7900 were designed with Primer Express (PE Biosystems) on the basis of the sequence information represented in SEQ ID NO:1, SEQ ID NO:3, SEQ ID NO:5, SEQ ID NO:7, SEQ ID NO:9 or SEQ ID NO:11. The primer for each gene is shown in Table 2.

**Table 2**

| | Forward primer | Reverse primer |
|---|---|---|
| RA2 | SEQ ID NO:25 | SEQ ID NO:26 |
| RA3 | SEQ ID NO:27 | SEQ ID NO:28 |
| DGPP1L, DGPP1S | SEQ ID NO:29 | SEQ ID NO:30 |
| DGPP2L, DGPP2S | SEQ ID NO:31 | SEQ ID NO:32 |
| G3DPH | SEQ ID NO:33 | SEQ ID NO:34 |

Total RNA (total RNA) was extracted from RA synovial tissues (G1 and G6 samples), OA synovial tissue (G7 sample), RA synovial fibroblast-like cells (RS1 and RS2 samples) and OA synovial fibroblast-like cells (OA1 and OA2 samples), using an RNA extraction reagent ISOGEN (Nippon Gene). The extraction was carried out according to the protocols attached to the reagent. The detail of the extraction method is now described below. To 100 mg of a frozen tissue, 1 ml of ISOGEN was added and homogenized, followed by standing for 5 minutes. After addition of 0.2 ml of chloroform (Kanto Chemical), the tissue was stirred and stood for 3 minutes. The tissue was centrifuged at 12,000 × g at 4°C for 15 minutes. The supernatant was recovered and 0.5 ml of 2-isopropanol (Kanto Chemical) was added thereto, followed by standing for 10 minutes. The mixture was then centrifuged at 12,000 × g at 4°C for 10 minutes. After the supernatant was removed, 1 ml of 70% ethanol was added. The resulting mixture was centrifuged at 7,500 × g at 4°C for 5 minutes. After drying in air, the product was dissolved in 50 µl of water.

The extracted total RNA was treated with DNase on a column, using a DNase processing kit (RNasey Mini kit, RNase-free DNase Set: both from Qiagen). According to the protocol attached to the kit, the treatment was carried out. The detail is described below. Water was added to the total RNA solution to become 100 µl. Subsequently, 350 ml of the buffer RLT contained in the kit was added and stirred. Then, 250 µl of ethanol was added and stirred, followed by charging in the column (RNeasy Mini spin column) included in the kit. The mixture was centrifuged at 8,000 × g for 15 seconds. After adding 350 µl of the buffer RW1 contained in the kit, centrifugation at 8,000 × g for 15 seconds was carried out. Further, 10 µl of the Dnase I stock solution and 70 µl of the buffer RDD contained in the kit were added, and the resulting mixture was left to stand at ambient temperature for 15 minutes. After adding 350 µl of the buffer RW1, centrifugation at 8,000 × g for 15 seconds was carried out. After adding 350 µl of the buffer RW1, centrifugation at 8,000 × g for 15 seconds was carried out. After adding 500 µl of the buffer RPE contained in the kit, centrifugation at 8,000 × g for 15 seconds was carried out. After adding 500 µl of the buffer RPE, centrifugation at 15,000 × g for 1 minute was carried out. After adding 30 µl of water, centrifugation at 8,000 × g for 1 minute was carried out to obtain the total RNA solution.

By reverse transcription with total RNA as template; cDNA was prepared. Total RNA (1 µg) was mixed with 10 µl of a random hexamer (100 ng/µl) (Amersham Pharmacia Biotech), and water was added thereto to become a final volume of 48 µl. After treatment at 70 °C for 10 minutes, the resulting mixture was placed on ice. Subsequently, 32 µl of an RT reaction mixture solution was added. The mixture solution had the following composition: 5 × First-Strand Buffer (250 mM Tris-HCl, pH 8.3, 375 mM KCl, 15 mM MgCl₂), 10 mM DTT, 0.5 mM dNTP, Superscript II RNase H⁻ reverse transcriptase (800 units) (all described above were from GIBCO BRL). After mixing, the resulting mixture was treated at 25°C for 15 minutes, 42°C for 50 minutes and 70°C for 15 minutes.

Using the prepared cDNA as template, the following experiments were carried out. As a template for preparing a standard curve for calculating the relative expression level of an object gene among each assay samples, a dilution series of human genomic DNA (Clontech) or a dilution series of a mixture solution of each assay samples was used. In order to correct the difference in cDNA concentration in samples, G3PDH gene as an internal standard for correction was similarly assayed and analyzed. Based on the expression level of the G3PDH gene, the expression level of an intended gene was corrected and calculated. Among the expression levels of the individual genes as corrected on a G3PDH basis, the expression level of each gene in the G7 sample from OA synovial tissue or the OA1 sample from synovial fibroblast-like cell was defined as 100 and the relative expression level of each gene in each sample was calculated. The results obtained by using synovial fibroblast-like cells are shown in Fig. 2 and Table 3. In G1 which is an RA synovial tissue sample compared with G7 which is an OA synovial tissue sample, expression levels of all the genes of RA2, RA3, RA4, DGPP1, and DGPP2 were apparently and significantly elevated. Additionally, it was found that compared with G1 with a larger inflammation level, the expression levels of the individual gene described above were significantly elevated among RA synovial tissues, and that the expression levels of the individual genes are elevated in relation with the score of pathological RA findings, namely that more exacerbated RA inflammation symptoms involve more elevation of the expression levels of the individual genes. When RS1 which is an RA synovial fibroblast-like sample compared with OA1 and OA2 which are OA synovial fibroblast-like samples, expression levels of all the genes of RA2, RA3, RA4, DGPP1 and DGPP2 were apparently and significantly elevated. When the RS1 sample among the RA synovial fibroblast-like samples compared with the RS2 sample, the expression levels of the individual genes were elevated significantly. It was found that the expression levels of the individual genes were elevated in relation with the score of pathological RA findings, namely that the severity of RA inflammation symptoms has a correlation with the elevation of the expression levels of the individual genes.

These indicate that RA progress can be detected by the methods described in the Example and detection of RA diagnosis is enabled.

**Table 3**

| | DGPP1 | DGPP2 | RA2 | RA3 |
|---|---|---|---|---|
| RS1 | 273 | 762 | 4042 | 2076 |
| RS2 | 246 | 221 | 1716 | 1136 |
| OA1 | 100 | 100 | 100 | 100 |
| OA2 | 86 | 123 | 66 | 99 |

### Industrial Applicability

It was shown that the polynucleotide of the present invention is an indicator for RA diagnosis since the elevation of the expression thereof is associated with the level of RA severity. The polynucleotide of the present invention, the antibody of the present invention and the oligonucleotide hybridizing with the polynucleotide of the present invention are useful for detection of RA diagnosis. The polypeptide of the present invention is useful for preparing the antibody of the present invention which is useful for such detection. Additionally, novel genes having enhanced expression in RA synovial cells are provided in accordance with the present invention and the novel genes can be applied to testing for RA diagnosis, by PCR using the specific primer sequence thereof.

### Sequence Listing Free Text

In the numerical title <223> in the Sequence Listing below, the "Artificial Sequence" is described. Specifically, individual nucleotide sequences of SEQ ID Nos.: 13 to 24 in the Sequence Listing are primer sequences artificially synthetized.

The present invention has been described above with reference to the specific embodiments. Variations and modifications thereof obvious to persons skilled in the art are also included within the scope of the present invention.

## Claims

(1) A polypeptide comprising an amino acid sequence represented by SEQ ID NO:2, SEQ ID NO:4, SEQ ID NO:6, SEQ ID NO:8 or SEQ ID NO:10 and having enhanced expression in chronic rheumatoid arthritis patients or
(2) a polypeptide comprising an amino acid sequence represented by SEQ ID NO:2, SEQ ID NO:4, SEQ ID NO:6, SEQ ID NO:8 or SEQ ID NO:10 in which 1 to 10 amino acids are substituted, deleted and/or inserted and having enhanced expression in chronic rheumatoid arthritis patients.

2. A polypeptide consisting of an amino acid sequence represented by SEQ ID NO:2, SEQ ID NO:4, SEQ ID NO:6, SEQ ID NO:8 or SEQ ID NO:10.

3. A polynucleotide encoding the polypeptide according to claim 1 or 2.

4. An expression vector comprising the polynucleotide according to claim 3.

5. A cell transformed with the expression vector according to claim 4.

6. A antibody which binds to a polypeptide comprising an amino acid sequence represented by SEQ ID NO:4 and having enhanced expression in chronic rheumatoid arthritis patients, a polypeptide comprising an amino acid sequence represented by SEQ ID NO:4 in which 1 to 10 amino acids are substituted, deleted and/or inserted and having enhanced expression in chronic rheumatoid arthritis patients or a polypeptide consisting of an amino acid sequence represented by SEQ ID NO:4.

7. A polynucleotide which specifically hybridizes to a polynucleotide represented by SEQ ID NO:1, SEQ ID NO:3, SEQ ID NO:5, SEQ ID NO:7 or SEQ ID NO:9 and has at least 15 nucleotides.

8. A method for detecting rheumatoid arthritis comprising
1) a step of assaying the expression level of a gene in a synovial biopsy sample of a test subject, said gene comprising
i) the nucleotide sequence according to claim 3 or
ii) a polynucleotide sequence encoding a polypeptide comprising an amino acid sequence represented by SEQ ID NO:12 and having enhanced expression in chronic rheumatoid arthritis patients, a polypeptide comprising an amino acid sequence represented by SEQ ID NO:12 in which 1 to 10 amino acids are substituted, deleted and/or inserted and having enhanced expression in chronic rheumatoid arthritis patients, or a polypeptide consisting of an amino acid sequence represented by SEQ ID NO:12; and
(2) a step of comparing the expression level of the gene with the expression levels thereof in a synovial biopsy sample in a normal subject or a patient with non-chronic rheumatoid arthritis.

9. A kit for detecting chronic rheumatoid arthritis comprising forward and reverse primers designed for enabling specific amplification of a gene represented by
1) the polynucleotide according to claim 3 or
2) a polynucleotide encoding a polypeptide comprising an amino acid sequence represented by SEQ ID NO:12 and having enhanced expression in chronic rheumatoid arthritis patients, a polypeptide comprising an amino acid sequence represented by SEQ ID NO:12 in which 1 to 10 amino acids are substituted, deleted and/or inserted and having enhanced expression in chronic rheumatoid arthritis patients, or a polypeptide consisting of an amino acid sequence represented by SEQ ID NO:12.


SEQUENCE LISTING

<110> Yamanouchi Pharmaceutical Co.,Ltd.
Harigai, Masayoshi

<120> RA relating novel gene

<130> YHA0332-PCT

<150> JP2002-211951
<151> 2002-07-22

<160> 34

<170> PatentIn version 3.1

<210> 1
<211> 1074
<212> DNA
<213> Homo sapiens

<220>
<221> CDS
<222> (1)..(1071)
<223> Inventor: Takeuchi, Masahiro; Yamaji, Noboru; Takasaki, Jun;
Akamatsu, Masahiko; Tsunoyama, Kazuhisa; Harigai, Masayoshi

<400> 1
atg cag ctc agg aat gtg tca gag caa gaa ctg gac agc gtg gcc atg 48
Met Gln Leu Arg Asn Val Ser Glu Gln Glu Leu Asp Ser Val Ala Met
1               5                   10                  15

aag ctc ctt cac caa gta agc aag ctg tgt ggg aag tgc agc ccc act 96
Lys Leu Leu His Gln Val Ser Lys Leu Cys Gly Lys Cys Ser Pro Thr
            20                  25                  30

gac gtg gac atc ctg cag ccc tcc ttc aac ttc ctg tat tgg agc ctt 144
Asp Val Asp Ile Leu Gln Pro Ser Phe Asn Phe Leu Tyr Trp Ser Leu
        35                  40                  45

cat cag acc aca ccc agc agt cag aaa aga gct gct gca gtg ctc ctg 192
His Gln Thr Thr Pro Ser Ser Gln Lys Arg Ala Ala Ala Val Leu Leu
    50                  55                  60

agc agc aca ggc ctg atg gag ctt ctg gag aag atg ctg gcc ctc acc 240
Ser Ser Thr Gly Leu Met Glu Leu Leu Glu Lys Met Leu Ala Leu Thr
65                  70                  75                  80

ttg gca aag gca gat tct ccc agg act gca ctc ctc tgc tct gcc tgg 288
Leu Ala Lys Ala Asp Ser Pro Arg Thr Ala Leu Leu Cys Ser Ala Trp
                85                  90                  95

ctg ctc act gcc tcc ttc tct gcc cag cag cac aag ggc agt ttg cag 336
Leu Leu Thr Ala Ser Phe Ser Ala Gln Gln His Lys Gly Ser Leu Gln
            100                 105                 110

gtt cac cag aca ctc tct gtg gaa atg gac caa gta ttg aag gct ctc 384
Val His Gln Thr Leu Ser Val Glu Met Asp Gln Val Leu Lys Ala Leu
        115                 120                 125

agc ttt cca aag aaa aag gct gca cta ctc tca gct gcc atc tta tgc 432
Ser Phe Pro Lys Lys Lys Ala Ala Leu Leu Ser Ala Ala Ile Leu Cys
    130                 135                 140

ttc ctg cgg aca gcc ctg cga caa agc ttt tcc tct gcc ctg gta gcc 480
Phe Leu Arg Thr Ala Leu Arg Gln Ser Phe Ser Ser Ala Leu Val Ala
145                 150                 155                 160

ctg gtg ccc tca ggg gcc cag cca ctg cca gcc acc aag gac act gtc 528
Leu Val Pro Ser Gly Ala Gln Pro Leu Pro Ala Thr Lys Asp Thr Val
                165                 170                 175

cta gct cca ctg cga atg tcg caa gtc cgg tcc ctg gtc att ggg ctg 576
Leu Ala Pro Leu Arg Met Ser Gln Val Arg Ser Leu Val Ile Gly Leu
            180                 185                 190

cag aac ctc ctg gtg cag aag gac cct cta ttg tcc cag gcc tgt gtt 624
Gln Asn Leu Leu Val Gln Lys Asp Pro Leu Leu Ser Gln Ala Cys Val
        195                 200                 205

ggc tgc ctg gag gcc ttg ctt gac tac ctg gat gcc cgg agc cca gac 672
Gly Cys Leu Glu Ala Leu Leu Asp Tyr Leu Asp Ala Arg Ser Pro Asp
    210                 215                 220

att gct ctc cac gtg gcc tcc cag cct tgg aat cgg ttt ttg ctg ttt 720
Ile Ala Leu His Val Ala Ser Gln Pro Trp Asn Arg Phe Leu Leu Phe
225                 230                 235                 240

acc ctc ttg gat gct gga gag aat tcc ttc ctc aga cct gag att ttg 768
Thr Leu Leu Asp Ala Gly Glu Asn Ser Phe Leu Arg Pro Glu Ile Leu
                245                 250                 255

agg ctc atg acc ctg ttt atg cgg tac cgg agt agc agt gtc ctc tct 816
Arg Leu Met Thr Leu Phe Met Arg Tyr Arg Ser Ser Ser Val Leu Ser
            260                 265                 270

cat gaa gag gtg ggt gat gtt ctg caa ggt gtg gct ttg gct gac ctg 864
His Glu Glu Val Gly Asp Val Leu Gln Gly Val Ala Leu Ala Asp Leu
        275                 280                 285

tct acc ctc tcg aac acc aca ctc cag gcc ctg cat ggc ttc ttc cag 912
Ser Thr Leu Ser Asn Thr Thr Leu Gln Ala Leu His Gly Phe Phe Gln
    290                 295                 300

cag ctc cag agc atg gga cac ctg gct gac cac agc atg gcc cag acc 960
Gln Leu Gln Ser Met Gly His Leu Ala Asp His Ser Met Ala Gln Thr
305                 310                 315                 320

ctg cag gcc tcc ttg gag ggc ctt ccc cct agc acc tcc tca ggc cag 1008
Leu Gln Ala Ser Leu Glu Gly Leu Pro Pro Ser Thr Ser Ser Gly Gln
                325                 330                 335

cca ccc ctg cag gac atg ctc tgc ctg gga ggg gtg gct gta tcc ctg 1056
Pro Pro Leu Gln Asp Met Leu Cys Leu Gly Gly Val Ala Val Ser Leu
            340                 345                 350

tcc cac atc aga aac tga 1074
Ser His Ile Arg Asn
        355

<210> 2
<211> 357
<212> PRT
<213> Homo sapiens

<400> 2

Met Gln Leu Arg Asn Val Ser Glu Gln Glu Leu Asp Ser Val Ala Met
1               5                   10                  15

Lys Leu Leu His Gln Val Ser Lys Leu Cys Gly Lys Cys Ser Pro Thr
            20                  25                  30

Asp Val Asp Ile Leu Gln Pro Ser Phe Asn Phe Leu Tyr Trp Ser Leu
        35                  40                  45

His Gln Thr Thr Pro Ser Ser Gln Lys Arg Ala Ala Ala Val Leu Leu
    50                  55                  60

Ser Ser Thr Gly Leu Met Glu Leu Leu Glu Lys Met Leu Ala Leu Thr
65                  70                  75                  80

Leu Ala Lys Ala Asp Ser Pro Arg Thr Ala Leu Leu Cys Ser Ala Trp
                85                  90                  95

Leu Leu Thr Ala Ser Phe Ser Ala Gln Gln His Lys Gly Ser Leu Gln
            100                 105                 110

Val His Gln Thr Leu Ser Val Glu Met Asp Gln Val Leu Lys Ala Leu
        115                 120                 125

Ser Phe Pro Lys Lys Lys Ala Ala Leu Leu Ser Ala Ala Ile Leu Cys
    130                 135                 140

Phe Leu Arg Thr Ala Leu Arg Gln Ser Phe Ser Ser Ala Leu Val Ala
145                 150                 155                 160

Leu Val Pro Ser Gly Ala Gln Pro Leu Pro Ala Thr Lys Asp Thr Val
                165                 170                 175

Leu Ala Pro Leu Arg Met Ser Gln Val Arg Ser Leu Val Ile Gly Leu
            180                 185                 190

Gln Asn Leu Leu Val Gln Lys Asp Pro Leu Leu Ser Gln Ala Cys Val
        195                 200                 205

Gly Cys Leu Glu Ala Leu Leu Asp Tyr Leu Asp Ala Arg Ser Pro Asp
    210                 215                 220

Ile Ala Leu His Val Ala Ser Gln Pro Trp Asn Arg Phe Leu Leu Phe
225                 230                 235                 240

Thr Leu Leu Asp Ala Gly Glu Asn Ser Phe Leu Arg Pro Glu Ile Leu
                245                 250                 255

Arg Leu Met Thr Leu Phe Met Arg Tyr Arg Ser Ser Ser Val Leu Ser
            260                 265                 270

His Glu Glu Val Gly Asp Val Leu Gln Gly Val Ala Leu Ala Asp Leu
        275                 280                 285

Ser Thr Leu Ser Asn Thr Thr Leu Gln Ala Leu His Gly Phe Phe Gln
    290                 295                 300

Gln Leu Gln Ser Met Gly His Leu Ala Asp His Ser Met Ala Gln Thr
305                 310                 315                 320

Leu Gln Ala Ser Leu Glu Gly Leu Pro Pro Ser Thr Ser Ser Gly Gln
                325                 330                 335

Pro Pro Leu Gln Asp Met Leu Cys Leu Gly Gly Val Ala Val Ser Leu
            340                 345                 350

Ser His Ile Arg Asn
        355

<210> 3
<211> 306
<212> DNA
<213> Homo sapiens

<220>
<221> CDS
<222> (1)..(303)
<223>

<400> 3
atg cct aga agg gga cca caa cag act cga cag gat cca ccg gtt ggg 48
Met Pro Arg Arg Gly Pro Gln Gln Thr Arg Gln Asp Pro Pro Val Gly
1               5                   10                  15

ccc aag gca gga gga agg gcg gcg ccc cca aac tcc cag gac gcc tgc 96
Pro Lys Ala Gly Gly Arg Ala Ala Pro Pro Asn Ser Gln Asp Ala Cys
            20                  25                  30

agc acc ccc cac gcg ccg ctc tcc gcc tct ggg gag cat cct gcc acc 144
Ser Thr Pro His Ala Pro Leu Ser Ala Ser Gly Glu His Pro Ala Thr
        35                  40                  45

ccc cga cac aca cac ccc ggc tac atc ccg cct tct cac gct tgg tca 192
Pro Arg His Thr His Pro Gly Tyr Ile Pro Pro Ser His Ala Trp Ser
    50                  55                  60

ggc gct ctg gag atg tcg gag atc cag gct ttt cct aaa gag tca gga 240
Gly Ala Leu Glu Met Ser Glu Ile Gln Ala Phe Pro Lys Glu Ser Gly
65                  70                  75                  80

ttg gaa ggc gga ctc cca ccg ttt gct gag ctc cac atg aca aca gca 288
Leu Glu Gly Gly Leu Pro Pro Phe Ala Glu Leu His Met Thr Thr Ala
                85                  90                  95

gac gac agg ccg cac tga 306
Asp Asp Arg Pro His
            100

<210> 4
<211> 101
<212> PRT
<213> Homo sapiens

<400> 4

Met Pro Arg Arg Gly Pro Gln Gln Thr Arg Gln Asp Pro Pro Val Gly
1               5                   10                  15

Pro Lys Ala Gly Gly Arg Ala Ala Pro Pro Asn Ser Gln Asp Ala Cys
            20                  25                  30

Ser Thr Pro His Ala Pro Leu Ser Ala Ser Gly Glu His Pro Ala Thr
        35                  40                  45

Pro Arg His Thr His Pro Gly Tyr Ile Pro Pro Ser His Ala Trp Ser
    50                  55                  60

Gly Ala Leu Glu Met Ser Glu Ile Gln Ala Phe Pro Lys Glu Ser Gly
65                  70                  75                  80

Leu Glu Gly Gly Leu Pro Pro Phe Ala Glu Leu His Met Thr Thr Ala
                85                  90                  95

Asp Asp Arg Pro His
            100

<210>  5
<211>  942
<212>  DNA
<213>  Homo sapiens

<220>
<221>  CDS
<222>  (1)..(939)
<223>

<400>  5
atg ccc tcg gca cag ccg cca ggc cgt ctt cct ggg gag ccg ccg gag        48
Met Pro Ser Ala Gln Pro Pro Gly Arg Leu Pro Gly Glu Pro Pro Glu
1               5                   10                  15

cgc ggg caa ctg gga gag ggg gcg gtg ccc agt tcc cgg ccc ggc cct        96
Arg Gly Gln Leu Gly Glu Gly Ala Val Pro Ser Ser Arg Pro Gly Pro
            20                  25                  30

ccc cgc gga ggt ggc cac gtc agc gca gcg tcg ctc gga gct cgc ggc       144
Pro Arg Gly Gly Gly His Val Ser Ala Ala Ser Leu Gly Ala Arg Gly
        35                  40                  45

cgg atg ggg aag gcg gcg gcg gcg gtg gcc ttt ggg gcc gaa gtg ggc       192
Arg Met Gly Lys Ala Ala Ala Ala Val Ala Phe Gly Ala Glu Val Gly
    50                  55                  60

gtg cgg ctc gcg ctg ttc gcg gcc ttc ctg gtg acg gag ctg ctc ccc       240

Val Arg Leu Ala Leu Phe Ala Ala Phe Leu Val Thr Glu Leu Leu Pro
65                  70                  75                  80

ccg ttc cag aga ctc atc cag ccg gag gag atg tgg ctc tac cgg aac      288
Pro Phe Gln Arg Leu Ile Gln Pro Glu Glu Met Trp Leu Tyr Arg Asn
                85                  90                  95

ccc tac gtg gag gcg gag tat ttc ccc acc aag ccg atg ttt gtt att      336
Pro Tyr Val Glu Ala Glu Tyr Phe Pro Thr Lys Pro Met Phe Val Ile
            100                 105                 110

gca ttt ctc tct cca ctg tct ctg atc ttc ctg gcc aaa ttt ctc aag      384
Ala Phe Leu Ser Pro Leu Ser Leu Ile Phe Leu Ala Lys Phe Leu Lys
        115                 120                 125

aag gca gac aca aga gac agc aga caa gcc tgc ctg gct gcc agc ctt      432
Lys Ala Asp Thr Arg Asp Ser Arg Gln Ala Cys Leu Ala Ala Ser Leu
    130                 135                 140

gcc ctg gct ctg aat ggc gtc ttt acc aac aca ata aaa ctg atc gta      480
Ala Leu Ala Leu Asn Gly Val Phe Thr Asn Thr Ile Lys Leu Ile Val
145                 150                 155                 160

ggg agg cca cgc cca gat ttc ttc tac cgc tgc ttc cct gat ggg cta      528
Gly Arg Pro Arg Pro Asp Phe Phe Tyr Arg Cys Phe Pro Asp Gly Leu
                165                 170                 175

gcc cat tct gac ttg atg tgt aca ggg gat aag gac gtg gtg aat gag      576
Ala His Ser Asp Leu Met Cys Thr Gly Asp Lys Asp Val Val Asn Glu
            180                 185                 190

ggc cga aag agc ttc ccc agt gga cat tct tcc ttt gca ttt gct ggt      624
Gly Arg Lys Ser Phe Pro Ser Gly His Ser Ser Phe Ala Phe Ala Gly
        195                 200                 205

ctg gcc ttt gcg tcc ttc tac ctg gca ggg aag tta cac tgc ttc aca      672

Leu Ala Phe Ala Ser Phe Tyr Leu Ala Gly Lys Leu His Cys Phe Thr
    210                 215                 220

cca caa ggc cgt ggg aaa tct tgg agg ttc tgt gcc ttt ctg tca cct 720
Pro Gln Gly Arg Gly Lys Ser Trp Arg Phe Cys Ala Phe Leu Ser Pro
225                 230                 235                 240

cta ctt ttt gca gct gtg att gca ctg tcc cgc aca tgt gac tac aag 768
Leu Leu Phe Ala Ala Val Ile Ala Leu Ser Arg Thr Cys Asp Tyr Lys
                245                 250                 255

cat cac tgg caa gat gta cta gtt gga tcc atg att gga atg aca ttt 816
His His Trp Gln Asp Val Leu Val Gly Ser Met Ile Gly Met Thr Phe
            260                 265                 270

gcc tat gtc tgc tat cgg cag tat tat cct cct ctg act gat gca gaa 864
Ala Tyr Val Cys Tyr Arg Gln Tyr Tyr Pro Pro Leu Thr Asp Ala Glu
        275                 280                 285

tgc cat aaa cca ttt caa gac aaa ctt gta ctt tcc act gca cag aag 912
Cys His Lys Pro Phe Gln Asp Lys Leu Val Leu Ser Thr Ala Gln Lys
    290                 295                 300

cct ggg gat tct tat tgt ttt gat att taa 942
Pro Gly Asp Ser Tyr Cys Phe Asp Ile
305                 310

<210> 6
<211> 313
<212> PRT
<213> Homo sapiens

<400> 6

Met Pro Ser Ala Gln Pro Pro Gly Arg Leu Pro Gly Glu Pro Pro Glu

1                5                   10                  15

Arg Gly Gln Leu Gly Glu Gly Ala Val Pro Ser Ser Arg Pro Gly Pro
            20                  25                  30

Pro Arg Gly Gly Gly His Val Ser Ala Ala Ser Leu Gly Ala Arg Gly
        35                  40                  45

Arg Met Gly Lys Ala Ala Ala Ala Val Ala Phe Gly Ala Glu Val Gly
    50                  55                  60

Val Arg Leu Ala Leu Phe Ala Ala Phe Leu Val Thr Glu Leu Leu Pro
65                  70                  75                  80

Pro Phe Gln Arg Leu Ile Gln Pro Glu Glu Met Trp Leu Tyr Arg Asn
                85                  90                  95

Pro Tyr Val Glu Ala Glu Tyr Phe Pro Thr Lys Pro Met Phe Val Ile
            100                 105                 110

Ala Phe Leu Ser Pro Leu Ser Leu Ile Phe Leu Ala Lys Phe Leu Lys
        115                 120                 125

Lys Ala Asp Thr Arg Asp Ser Arg Gln Ala Cys Leu Ala Ala Ser Leu
    130                 135                 140

Ala Leu Ala Leu Asn Gly Val Phe Thr Asn Thr Ile Lys Leu Ile Val

145                 150                 155                 160

Gly Arg Pro Arg Pro Asp Phe Phe Tyr Arg Cys Phe Pro Asp Gly Leu
                165                 170                 175

Ala His Ser Asp Leu Met Cys Thr Gly Asp Lys Asp Val Val Asn Glu
            180                 185                 190

Gly Arg Lys Ser Phe Pro Ser Gly His Ser Ser Phe Ala Phe Ala Gly
        195                 200                 205

Leu Ala Phe Ala Ser Phe Tyr Leu Ala Gly Lys Leu His Cys Phe Thr
    210                 215                 220

Pro Gln Gly Arg Gly Lys Ser Trp Arg Phe Cys Ala Phe Leu Ser Pro
225                 230                 235                 240

Leu Leu Phe Ala Ala Val Ile Ala Leu Ser Arg Thr Cys Asp Tyr Lys
                245                 250                 255

His His Trp Gln Asp Val Leu Val Gly Ser Met Ile Gly Met Thr Phe
            260                 265                 270

Ala Tyr Val Cys Tyr Arg Gln Tyr Tyr Pro Pro Leu Thr Asp Ala Glu
        275                 280                 285

Cys His Lys Pro Phe Gln Asp Lys Leu Val Leu Ser Thr Ala Gln Lys

290                 295                 300

Pro Gly Asp Ser Tyr Cys Phe Asp Ile
305                 310

<210>  7
<211>  795
<212>  DNA
<213>  Homo sapiens

<220>
<221>  CDS
<222>  (1)..(792)
<223>

<400>  7
atg ggg aag gcg gcg gcg gcg gtg gcc ttt ggg gcc gaa gtg ggc gtg        48
cgg ctc gcg ctg ttc gcg gcc ttc ctg gtg acg gag ctg ctc ccc ccg        96
ttc cag aga ctc atc cag ccg gag gag atg tgg ctc tac cgg aac ccc       144
tac gtg gag gcg gag tat ttc ccc acc aag ccg atg ttt gtt att gca       192
ttt ctc tct cca ctg tct ctg atc ttc ctg gcc aaa ttt ctc aag aag       240
gca gac aca aga gac agc aga caa gcc tgc ctg gct gcc agc ctt gcc      288
ctg gct ctg aat ggc gtc ttt acc aac aca ata aaa ctg atc gta ggg      336
agg cca cgc cca gat ttc ttc tac cgc tgc ttc cct gat ggg cta gcc      384
cat tct gac ttg atg tgt aca ggg gat aag gac gtg gtg aat gag ggc      432
cga aag agc ttc ccc agt gga cat tct tcc ttt gca ttt gct ggt ctg      480
gcc ttt gcg tcc ttc tac ctg gca ggg aag tta cac tgc ttc aca cca      528
caa ggc cgt ggg aaa tct tgg agg ttc tgt gcc ttt ctg tca cct cta      576
ctt ttt gca gct gtg att gca ctg tcc cgc aca tgt gac tac aag cat      624
cac tgg caa gat gta cta gtt gga tcc atg att gga atg aca ttt gcc      672
tat gtc tgc tat cgg cag tat tat cct cct ctg act gat gca gaa tgc        720
cat aaa cca ttt caa gac aaa ctt gta ctt tcc act gca cag aag cct        768
ggg gat tct tat tgt ttt gat att taa                                    795

<210>  8
<211>  264
<212>  PRT
<213>  Homo sapiens

<400>  8

<210>  9
<211>  876
<212>  DNA
<213>  Homo sapiens

<220>
<221>  CDS
<222>  (1)..(873)
<223>

<400>  9
atg gct gcg gga gcc gcg gag agc acc agc tgt cgc cgc ggg agc tgc        48
Met Ala Ala Gly Ala Ala Glu Ser Thr Ser Cys Arg Arg Gly Ser Cys
1               5                   10                  15

tcc ggc cgc acc atg cgg gag ctg gcc att gag atc ggg gtg cga gcc        96
Ser Gly Arg Thr Met Arg Glu Leu Ala Ile Glu Ile Gly Val Arg Ala
            20                  25                  30

ctg ctc ttc gga gtc ttc gtt ttt aca gag ttt ttg gat ccg ttc cag 144
Leu Leu Phe Gly Val Phe Val Phe Thr Glu Phe Leu Asp Pro Phe Gln
        35                  40                  45

aga gtc atc cag cca gaa gag atc tgg ctc tat aaa aat cct ttg gtg 192
Arg Val Ile Gln Pro Glu Glu Ile Trp Leu Tyr Lys Asn Pro Leu Val
    50                  55                  60

caa tca gat aac ata cct acc cgc ctc atg ttt gca att tct ttc ctc 240
Gln Ser Asp Asn Ile Pro Thr Arg Leu Met Phe Ala Ile Ser Phe Leu
65                  70                  75                  80

aca ccc ctg gct gtt att tgt gtg gtg aaa att atc cgg cga aca gac 288
Thr Pro Leu Ala Val Ile Cys Val Val Lys Ile Ile Arg Arg Thr Asp
                85                  90                  95

aag act gaa att aag gaa gcc ttc tta gcg gtg tcc ttg gct ctt gct 336
Lys Thr Glu Ile Lys Glu Ala Phe Leu Ala Val Ser Leu Ala Leu Ala
            100                 105                 110

ttg aat gga gtc tgc aca aac act att aaa tta ata gtg gga aga cct 384
Leu Asn Gly Val Cys Thr Asn Thr Ile Lys Leu Ile Val Gly Arg Pro
        115                 120                 125

cgc ccc gat ttc ttt tac cgc tgc ttt cca gat gga gtg atg aac tcg 432
Arg Pro Asp Phe Phe Tyr Arg Cys Phe Pro Asp Gly Val Met Asn Ser
    130                 135                 140

gaa atg cat tgc aca ggt gac ccc gat ctg gtg tcc gag ggc cgc aaa 480
Glu Met His Cys Thr Gly Asp Pro Asp Leu Val Ser Glu Gly Arg Lys
145                 150                 155                 160

agc ttc ccc agc atc cat tcc tcc ttt gcc ttt tcg ggc ctt ggc ttc 528
Ser Phe Pro Ser Ile His Ser Ser Phe Ala Phe Ser Gly Leu Gly Phe
                165                 170                 175

acg acg ttc tac ttg gcg ggc aag ctg cac tgc ttc acc gag agt ggg 576
Thr Thr Phe Tyr Leu Ala Gly Lys Leu His Cys Phe Thr Glu Ser Gly
            180                 185                 190

cgg gga aag agc tgg cgg ctc tgt gct gcc atc ctg ccc ttg tac tgc 624
Arg Gly Lys Ser Trp Arg Leu Cys Ala Ala Ile Leu Pro Leu Tyr Cys
        195                 200                 205

gcc atg atg att gcc ctg tcc cgc atg tgc gac tac aag cat cac tgg 672
Ala Met Met Ile Ala Leu Ser Arg Met Cys Asp Tyr Lys His His Trp
    210                 215                 220

caa gat tcc ttt gtg ggt gga gtc atc ggc ctc att ttt gca tac att 720
Gln Asp Ser Phe Val Gly Gly Val Ile Gly Leu Ile Phe Ala Tyr Ile
225                 230                 235                 240

tgc tac aga cag cac tat cct cct ctg gcc aac aca gct tgc cat aaa 768
Cys Tyr Arg Gln His Tyr Pro Pro Leu Ala Asn Thr Ala Cys His Lys
                245                 250                 255

ccc tac gtt agt ctg cga gtc cca gcc tca ctg aag aaa gag gag agg 816
Pro Tyr Val Ser Leu Arg Val Pro Ala Ser Leu Lys Lys Glu Glu Arg
            260                 265                 270

ccc aca gct gac agc gca ccc agc ttg cct ctg gag ggg atc acc gaa 864
Pro Thr Ala Asp Ser Ala Pro Ser Leu Pro Leu Glu Gly Ile Thr Glu
        275                 280                 285

ggc ccg gta tga 876
Gly Pro Val
    290

<210> 10
<211> 291

<212>  PRT
<213>  Homo sapiens

<400>  10

Met Ala Ala Gly Ala Ala Glu Ser Thr Ser Cys Arg Arg Gly Ser Cys
1               5                   10                  15

Ser Gly Arg Thr Met Arg Glu Leu Ala Ile Glu Ile Gly Val Arg Ala
            20                  25                  30

Leu Leu Phe Gly Val Phe Val Phe Thr Glu Phe Leu Asp Pro Phe Gln
        35                  40                  45

Arg Val Ile Gln Pro Glu Glu Ile Trp Leu Tyr Lys Asn Pro Leu Val
    50                  55                  60

Gln Ser Asp Asn Ile Pro Thr Arg Leu Met Phe Ala Ile Ser Phe Leu
65                  70                  75                  80

Thr Pro Leu Ala Val Ile Cys Val Val Lys Ile Ile Arg Arg Thr Asp
                85                  90                  95

Lys Thr Glu Ile Lys Glu Ala Phe Leu Ala Val Ser Leu Ala Leu Ala
            100                 105                 110

Leu Asn Gly Val Cys Thr Asn Thr Ile Lys Leu Ile Val Gly Arg Pro
        115                 120                 125

Arg Pro Asp Phe Phe Tyr Arg Cys Phe Pro Asp Gly Val Met Asn Ser
    130                 135                 140

Glu Met His Cys Thr Gly Asp Pro Asp Leu Val Ser Glu Gly Arg Lys
145                 150                 155                 160

Ser Phe Pro Ser Ile His Ser Ser Phe Ala Phe Ser Gly Leu Gly Phe
                165                 170                 175

Thr Thr Phe Tyr Leu Ala Gly Lys Leu His Cys Phe Thr Glu Ser Gly
            180                 185                 190

Arg Gly Lys Ser Trp Arg Leu Cys Ala Ala Ile Leu Pro Leu Tyr Cys
        195                 200                 205

Ala Met Met Ile Ala Leu Ser Arg Met Cys Asp Tyr Lys His His Trp
    210                 215                 220

Gln Asp Ser Phe Val Gly Gly Val Ile Gly Leu Ile Phe Ala Tyr Ile
225                 230                 235                 240

Cys Tyr Arg Gln His Tyr Pro Pro Leu Ala Asn Thr Ala Cys His Lys
                245                 250                 255

Pro Tyr Val Ser Leu Arg Val Pro Ala Ser Leu Lys Lys Glu Glu Arg
            260                 265                 270

Pro Thr Ala Asp Ser Ala Pro Ser Leu Pro Leu Glu Gly Ile Thr Glu
        275                 280                 285

Gly Pro Val
    290

<210> 11
<211> 816
<212> DNA
<213> Homo sapiens

<220>
<221> CDS
<222> (1)..(813)
<223>

<400> 11
atg cgg gag ctg gcc att gag atc ggg gtg cga gcc ctg ctc ttc gga        48
Met Arg Glu Leu Ala Ile Glu Ile Gly Val Arg Ala Leu Leu Phe Gly
1               5                   10                  15

gtc ttc gtt ttt aca gag ttt ttg gat ccg ttc cag aga gtc atc cag        96
Val Phe Val Phe Thr Glu Phe Leu Asp Pro Phe Gln Arg Val Ile Gln
            20                  25                  30

cca gaa gag atc tgg ctc tat aaa aat cct ttg gtg caa tca gat aac       144
Pro Glu Glu Ile Trp Leu Tyr Lys Asn Pro Leu Val Gln Ser Asp Asn
        35                  40                  45

ata cct acc cgc ctc atg ttt gca att tct ttc ctc aca ccc ctg gct       192
Ile Pro Thr Arg Leu Met Phe Ala Ile Ser Phe Leu Thr Pro Leu Ala
    50                  55                  60

gtt att tgt gtg gtg aaa att atc cgg cga aca gac aag act gaa att 240
Val Ile Cys Val Val Lys Ile Ile Arg Arg Thr Asp Lys Thr Glu Ile
65                  70                  75                  80

aag gaa gcc ttc tta gcg gtg tcc ttg gct ctt gct ttg aat gga gtc 288
Lys Glu Ala Phe Leu Ala Val Ser Leu Ala Leu Ala Leu Asn Gly Val
                85                  90                  95

tgc aca aac act att aaa tta ata gtg gga aga cct cgc ccc gat ttc 336
Cys Thr Asn Thr Ile Lys Leu Ile Val Gly Arg Pro Arg Pro Asp Phe
            100                 105                 110

ttt tac cgc tgc ttt cca gat gga gtg atg aac tcg gaa atg cat tgc 384
Phe Tyr Arg Cys Phe Pro Asp Gly Val Met Asn Ser Glu Met His Cys
        115                 120                 125

aca ggt gac ccc gat ctg gtg tcc gag ggc cgc aaa agc ttc ccc agc 432
Thr Gly Asp Pro Asp Leu Val Ser Glu Gly Arg Lys Ser Phe Pro Ser
    130                 135                 140

atc cat tcc tcc ttt gcc ttt tcg ggc ctt ggc ttc acg acg ttc tac 480
Ile His Ser Ser Phe Ala Phe Ser Gly Leu Gly Phe Thr Thr Phe Tyr
145                 150                 155                 160

ttg gcg ggc aag ctg cac tgc ttc acc gag agt ggg cgg gga aag agc 528
Leu Ala Gly Lys Leu His Cys Phe Thr Glu Ser Gly Arg Gly Lys Ser
                165                 170                 175

tgg cgg ctc tgt gct gcc atc ctg ccc ttg tac tgc gcc atg atg att 576
Trp Arg Leu Cys Ala Ala Ile Leu Pro Leu Tyr Cys Ala Met Met Ile
            180                 185                 190

gcc ctg tcc cgc atg tgc gac tac aag cat cac tgg caa gat tcc ttt 624
Ala Leu Ser Arg Met Cys Asp Tyr Lys His His Trp Gln Asp Ser Phe
        195                 200                 205

gtg ggt gga gtc atc ggc ctc att ttt gca tac att tgc tac aga cag 672
Val Gly Gly Val Ile Gly Leu Ile Phe Ala Tyr Ile Cys Tyr Arg Gln
    210                 215                 220

cac tat cct cct ctg gcc aac aca gct tgc cat aaa ccc tac gtt agt 720
His Tyr Pro Pro Leu Ala Asn Thr Ala Cys His Lys Pro Tyr Val Ser
225                 230                 235                 240

ctg cga gtc cca gcc tca ctg aag aaa gag gag agg ccc aca gct gac 768
Leu Arg Val Pro Ala Ser Leu Lys Lys Glu Glu Arg Pro Thr Ala Asp
                245                 250                 255

agc gca ccc agc ttg cct ctg gag ggg atc acc gaa ggc ccg gta tga 816
Ser Ala Pro Ser Leu Pro Leu Glu Gly Ile Thr Glu Gly Pro Val
            260                 265                 270

<210> 12
<211> 271
<212> PRT
<213> Homo sapiens

<400> 12

Met Arg Glu Leu Ala Ile Glu Ile Gly Val Arg Ala Leu Leu Phe Gly
1               5                   10                  15

Val Phe Val Phe Thr Glu Phe Leu Asp Pro Phe Gln Arg Val Ile Gln
            20                  25                  30

Pro Glu Glu Ile Trp Leu Tyr Lys Asn Pro Leu Val Gln Ser Asp Asn
        35                  40                  45

Ile Pro Thr Arg Leu Met Phe Ala Ile Ser Phe Leu Thr Pro Leu Ala
    50                  55                  60

Val Ile Cys Val Val Lys Ile Ile Arg Arg Thr Asp Lys Thr Glu Ile
65                  70                  75                  80

Lys Glu Ala Phe Leu Ala Val Ser Leu Ala Leu Ala Leu Asn Gly Val
                85                  90                  95

Cys Thr Asn Thr Ile Lys Leu Ile Val Gly Arg Pro Arg Pro Asp Phe
            100                 105                 110

Phe Tyr Arg Cys Phe Pro Asp Gly Val Met Asn Ser Glu Met His Cys
        115                 120                 125

Thr Gly Asp Pro Asp Leu Val Ser Glu Gly Arg Lys Ser Phe Pro Ser
    130                 135                 140

Ile His Ser Ser Phe Ala Phe Ser Gly Leu Gly Phe Thr Thr Phe Tyr
145                 150                 155                 160

Leu Ala Gly Lys Leu His Cys Phe Thr Glu Ser Gly Arg Gly Lys Ser
                165                 170                 175

Trp Arg Leu Cys Ala Ala Ile Leu Pro Leu Tyr Cys Ala Met Met Ile
            180                 185                 190

Ala Leu Ser Arg Met Cys Asp Tyr Lys His His Trp Gln Asp Ser Phe
        195                 200                 205

Val Gly Gly Val Ile Gly Leu Ile Phe Ala Tyr Ile Cys Tyr Arg Gln
    210                 215                 220

His Tyr Pro Pro Leu Ala Asn Thr Ala Cys His Lys Pro Tyr Val Ser
225                 230                 235                 240

Leu Arg Val Pro Ala Ser Leu Lys Lys Glu Glu Arg Pro Thr Ala Asp
                245                 250                 255

Ser Ala Pro Ser Leu Pro Leu Glu Gly Ile Thr Glu Gly Pro Val
            260                 265                 270

<210>  13
<211>  37
<212>  DNA
<213>  Artificial

<220>
<223>  Description of Artificial Sequence:an artificially
       synthesized primer sequence

<400>  13
cgcgcggatc cgccaccatg cagctcagga atgtgtc                                37

<210>  14
<211>  31
<212>  DNA

<213>  Artificial

<220>
<223>  Description of Artificial Sequence:an artificially
        synthesized primer sequence

<400>  14
gcgcgctcga ggtttctgat gtgggacagg g                                    31


<210>  15
<211>  37
<212>  DNA
<213>  Artificial

<220>
<223>  Description of Artificial Sequence:an artificially
        synthesized primer sequence

<400>  15
gcgcgaagct tgccaccatg cctagaaggg gaccaca                              37


<210>  16
<211>  30
<212>  DNA
<213>  Artificial

<220>
<223>  Description of Artificial Sequence:an artificially
        synthesized primer sequence

<400>  16
gcgcgctcga ggtgcggcct gtcgtctgct                                      30

<210> 17
<211> 35
<212> DNA
<213> Artificial

<220>
<223> Description of Artificial Sequence:an artificially
synthesized primer sequence

<400> 17
gcgcgaagct tgccaccatg ccctcggcac agccg 35

<210> 18
<211> 35
<212> DNA
<213> Artificial

<220>
<223> Description of Artificial Sequence:an artificially
synthesized primer sequence

<400> 18
gcgcgctcga gaatatcaaa acaataagaa tcccc 35

<210> 19
<211> 35
<212> DNA
<213> Artificial

<220>
<223> Description of Artificial Sequence:an artificially
synthesized primer sequence

<400> 19

gcgcgaagct tgccaccatg gggaaggcgg cggcg                                35

<210>  20
<211>  35
<212>  DNA
<213>  Artificial

<220>
<223>  Description of Artificial Sequence:an artificially
       synthesized primer sequence

<400>  20
gcgcgaagct tgccaccatg gctgcgggag ccgcg                                35

<210>  21
<211>  30
<212>  DNA
<213>  Artificial

<220>
<223>  Description of Artificial Sequence:an artificially
       synthesized primer sequence

<400>  21
gcgcgctcga gtaccgggcc ttcggtgatc                                      30

<210>  22
<211>  36
<212>  DNA
<213>  Artificial

<220>
<223>  Description of Artificial Sequence:an artificially

synthesized primer sequence

<400> 22
gcgcgaagct tgccaccatg cgggagctgg ccattg 36

<210> 23
<211> 31
<212> DNA
<213> Artificial

<220>
<223> Description of Artificial Sequence:an artificially
synthesized primer sequence

<400> 23
tcgaggacta caaggacgac gatgacaagc t 31

<210> 24
<211> 31
<212> DNA
<213> Artificial

<220>
<223> Description of Artificial Sequence:an artificially
synthesized primer sequence

<400> 24
ctagagcttg tcatcgtcgt ccttgtagtc c 31

<210> 25
<211> 16
<212> DNA
<213> Homo sapiens

<400> 25
cacgtggcct cccagc 16

<210> 26
<211> 24
<212> DNA
<213> Homo sapiens

<400> 26
taggttctcc catttgtcgt tttt 24

<210> 27
<211> 20
<212> DNA
<213> Homo sapiens

<400> 27
cccttgaaca acgcaggttc 20

<210> 28
<211> 20
<212> DNA
<213> Homo sapiens

<400> 28
tttgtaggga cacccacctg 20

<210> 29
<211> 21
<212> DNA
<213> Homo sapiens

<400> 29
cttccaaggt gcaagtgagg a 21

<210> 30
<211> 23
<212> DNA
<213> Homo sapiens

<400> 30
cattggaggc agaatacagt gtg 23

<210> 31
<211> 20
<212> DNA
<213> Homo sapiens

<400> 31
tcctgggagg atggacacta 20

<210> 32
<211> 21
<212> DNA
<213> Homo sapiens

<400> 32
tgatgtcagg gtggcagatg t 21

<210> 33
<211> 18
<212> DNA
<213> Homo sapiens

<400> 33
gggaaggtga aggtcgga 18

<210> 34
<211> 17
<212> DNA
<213> Homo sapiens

<400> 34
gcagccctgg tgaccag 17